# EUROPÄISCHE PATENTANMELDUNG

(11) **EP 0 697 216 A2**
(43) Veröffentlichungstag der Anmeldung: **21.02.1996**
(21) Anmeldenummer: 95111739.9
(22) Anmeldetag: 26.07.1995
(51) Int. Cl.: A61L 9/12, A61L 9/01

(54) **Natürliche Lufterfrischer**

(30) Priorität: 26.07.1994 CH 2359/94
(71) Anmelder: Stricker, Egon, CH-8340 Hadlikon (CH)
(72) Erfinder: Die Erfindernennung liegt noch nicht vor
(74) Vertreter: Blum, Rudolf Emil Ernst

(57) **Zusammenfassung**

Es wird ein in Richtung der natürlichen Luftströmung durchströmbarer Lufterfrischer sowie ein natürliches, geruchsbindendes Mittel beschrieben, das insbesonders auch zur Verwendung im durchströmbaren Lufterfrischer geeignet ist und auch zum Entfernen von Ungeziefer verwendet werden kann.

## Beschreibung

Die vorliegende Erfindung betrifft Lufterfrischer, insbesonders Lufterfrischer auf der Basis natürlicher Materialien.

Das Auftreten unangenehmer Düfte im Wohn-, Küchen- und Toilettenbereich ist ein altbekanntes Problem, zu dessen Lösung bereits viele Anstrengungen unternommen worden sind.

Zum Beispiel sind Aktivkohlefilter zur Geruchsbindung in Kühlschränken seit langem im Handel.

Im Wohn- und Toilettenbereich kommen insbesonders Produkte zum Einsatz die einen angenehmen Duft abgeben, wie Sprays und Duftlampen.

Ziel der vorliegenden Erfindung war es, einen Lufterfrischer bereitzustellen, der auf natürlicher Basis effizient die Luft durch Binden unerwünschter Geruchstoffe verbessert und gegebenenfalls einen angenehmen Duft abgibt.

Dieses Ziel wurde erreicht durch Bereitstellung von Lufterfrischern, die meistens in der Hauptrichtung der Luftbewegung durchströmbar sind sowie verbesserter Sorptionsmittel. Der Begriff Sorptionsmittel, wie er hier in der Folge gebraucht wird, umfasst wahlweise Ad-, Ab- und Chemisorptionsmittel oder deren Kombination.

Die gegenwärtig insbesonders in Kühlschränken verwendeten Lufterfrischer sind derart konstruiert, dass das Sorptionsmittel zwar in möglichst direktem Kontakt mit der Raumluft steht, es ist jedoch kaum Durchströmung vorgesehen, da die Produkte des Standes der Technik in Strömungsrichtung geschlossen sind.

Der erfindungsgemässe Lufterfrischer ermöglicht es nun, die natürliche Luftbewegung, d.h. den Luftaustausch durch Absinken der kalten und Steigen der warmen Luft zur Durchströmung des Lufterfrischers auszunützen. Entsprechende Luftströme treten auch z.B. in Kühlschränken auf.

Es ist deshalb wünschenswert, dass der Lufterfrischer insbesonders in Richtung der Luftbewegung optimal durchströmbar ist, bei üblicherweise senkrechter Luftbewegung, also in senkrechter Richtung.

Ein solcher Lufterfrischer kann z.B. aus mindestens einem entsprechend geformten Stück des Sorptionsmittels bestehen, z.B. einem nicht allzu dichten Presskohlestück, oder er kann aus mindestens einem porösen Stoff wie Schaumstoff, Fliesstoff bestehen, an dessen Oberfläche ein Sorptionsmittel aufgebracht ist, z.B. mittels eines Haftmittels. Ein solcher Lufterfrischer ist vorzugsweise mit einer Aufhängevorrichtung, z.B. einem Loch zum Durchziehen eines Drahtes, versehen.

Es ist jedoch stark bevorzugt, dass der Lufterfrischer zusätzlich einen Behälter aufweist. Der erfindungsgemässe Behälter ist derart konstruiert, dass er das Sorptionsmittel schützt und dennoch die Durchströmung zulässt. Zudem kann bei der Verwendung eines Behälters feines Sorptionsmittel, d.h. mit grösserer Oberfläche, eingesetzt werden.

Figur 1 zeigt einen an einer Kante aufhängbaren, allseitig durchströmbaren Würfel mit unterschiedlicher Ausführungsform der Durchströmungsflächen.

Figur 2 zeigt einen auf einer Schmalseite aufstellbaren, ebenfalls allseitig durchströmbaren Quader, ebenfalls mit unterschiedlicher Ausführungsform der Flächen.

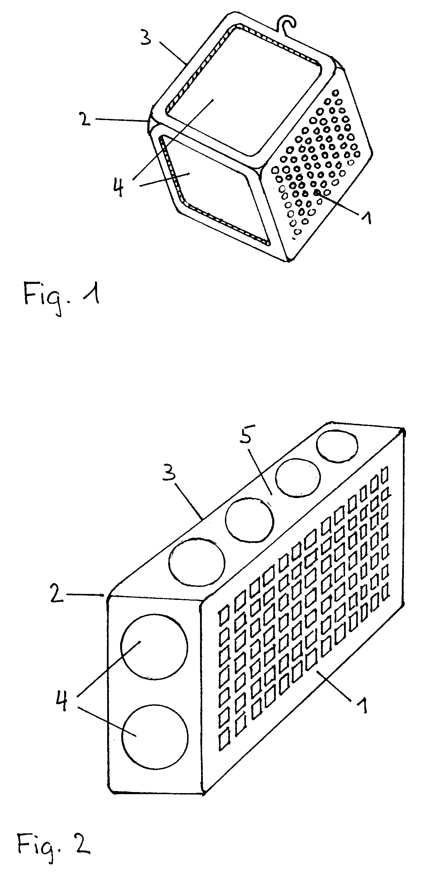

Speziell gute Durchströmbarkeit wurde nun bei einem Behälter in Form eines vorzugsweise an einer Ecke oder speziell bevorzugt einer Kante aufgehängten resp. auf einer Ecke oder insbesonders auf einer Kante stehenden Würfel gefunden, dessen sämtliche Flächen durchströmbar sind, so dass auch kurzfristige Ablenkungen der Ströme wie sie z.B. beim Oeffnen resp. Schliessen des Kühlschrankes entstehen, erfasst werden. Durch die spezielle auf einer Kante stehende resp. an einer Kante hängende Anordnung wird zudem vermieden, dass die Durchströmung durch Flächenkontakt mit einer Wand behindert werden kann. Eine mögliche Ausführungsform ist in Figur 1 dargestellt.

Der Behälter in dieser Ausführungsform weist einen spritzgegossenen Körper auf, der zwei gegenüberliegende durchströmbare Seiten 1 sowie die Ecken 2 und Kanten 3 der weiteren vier Seiten des Würfels bildet, wodurch grosse Durchströmungsöffnungen 4 entstehen. Diese Oeffnungen 4 können mit durchströmbarem Material, z.B. Schaumstoff oder Filterstoff derart verschlossen werden, dass auch feines Sorptionsmaterial zurückgehalten wird.

Für die Aufstellung auf einer Kante kann diese entweder abgeflacht sein oder ein, vorzugsweise durchströmbarer, Fuss (z.B. eine Querstrebe) vorgesehen werden.

Eine andere vorteilhafte Form ist ein Quader (vgl. Fig. 2), der ebenfalls allseitig durchströmbar ist. Ein solcher Quader kann leicht so ausgerichtet werden, dass die Durchströmungsstrecke und damit die Kontaktzeit der durchströmenden Luft mit dem Sorptionsmittel in Hauptströmungsrichtung länger ist als in den Nebenströmungsrichtungen. Im Kühlschrank wird ein quaderförmiger Körper z.B. vorzugsweise auf einer seiner vier Schmalseiten aufgestellt resp. in entsprechender Position aufgehängt.

Der in Figur 2 dargestellte Behälter weist einen spritzgegossenen Kunststoffkörper auf, der eine gitterförmige Fläche 1 sowie die Ecken 2, Kanten 3 und die Unterteilung 5 der Durchströmungsöffnungen 4 bildet. Je nach Grösse der Durchströmungsöffnungen sind diese mit durchströmbarem Material verschlossen, um feines Sorptionsmittel zurückzuhalten.

Der Behälter weist allgemein vorzugsweise ein spritzgegossenes Kunststoffgehäuse auf.

Die durchströmbaren Wände 1 sollten das Sorptionsmittel möglichst vollständig zurückhalten. Deshalb können diese entweder im Spritzgussverfahren direkt herstellbare mehr oder weniger feine Siebe sein oder aber grosse Oeffnungen 4 aufweisen, die vor dem Einfüllen des Sorptionsmittels mit porösem Material wie Filtermatten verschlossen werden.

Alternativ kann selbstverständlich auch das Sorptionsmittel selbst in eine poröse Hülle verpackt eingebracht werden, z.B. eingeschweisst in eine Filterfolie.

Der erfindungsgemässe Lufterfrischer kann mehr als ein Sorptionsmittel enthalten oder aus mehr als einem Sorptionsmittel bestehen. Insbesondere der Behälter kann mehrere Kammern zur Aufnahme verschiedener Sorptionsmittel aufweisen.

Der erfindungsgemässe Lufterfrischer enthält natürliche Sorptionsmittel in vorzugsweise einem durchströmbaren Behälter, wie oben beschrieben.

Als Sorptionsmittel geeignet sind einerseits aktivierte Holzkohle, die allgemein als Aktivkohle bezeichnet wird. Diese wird aus handhabungstechnischen Gründen insbesonders in verpresster Form eingesetzt.

Aktivkohle ist als Sorptionsmittel in Kühlschränken bereits bekannt.

Es wurde nun gefunden, dass Zedernholz, insbesonders das Holz der "Eastern aromatic red cedar" nicht nur ein Mottenschutz und in gewissem Masse feuchtigkeitsabsorbierend ist, sondern auch geruchsbindend wirkt, wobei es gleichzeitig einen angenehmen Duft ausströmt und alleine, sowie insbesondere auch zusammen mit Aktivkohle, zu einer starken Verbesserung der Luftqualität beiträgt.

Zedernholz kann in beliebiger Form und Grösse vorliegen, es wird aber vorteilhafterweise in verkleinerter Form, z.B. als Pulver, Sägespähne oder Holzhobel verwendet, d.h. in einer Form mit grosser Oberfläche. Ein Vorteil von Zedernholz als Adsorptionsmittel ist, dass es auch in Form eines Gebrauchsgegenstandes, wie einer Schale oder einem Kleiderbügel, verwendet werden kann und wirksam ist.

Bei Verwendung von flexiblen Behältern sind Zedernholzspähne den Aktivkohlepresslingen vorzuziehen, da diese weniger reibungsempfindlich sind.

Zedernholz, insbesonders in Form von Spähnen ist zudem ein äusserst geeigneter geruchsbindender Zusatz zu Katzenstreu.

Es hat sich zudem gezeigt, dass Zedernholz und/oder Zedernöl keineswegs nur gegen Kleidermotten wirkt. Vielmehr kann es auch als natürliches Mittel gegen Flöhe und Zecken, z.B. als Zusatz zu Katzenstreu eingesetzt werden, resp. in Räumen und Schränken allgemein auch gegen Stechmücken und Küchenschaben.

Speziell wirksam auch in bezug auf Ungeziefer ist das Holz der in den USA heimischen "Eastern Aromatic Red Cedar". Dieses wirkt z.B. nicht primär mottenvertreibend wie dasjenige der Atlaszeder sondern im Gegenteil mottenanziehend aber larvenabtötend.

Anstelle von Zedernholz selbst können für gewisse Verwendungen, insbesondere zum Entfernen von Ungeziefer, auch Zedernöl resp. mit Zedernöl imprägnierte Gegenstände, wie Duftsteine, verwendet werden.

Neben Aktivkohle kann Zedernholz auch mit anderen Materialien gemischt werden, insbesonders mit natürlichen Insektiziden wie Nelken und Lavendel resp. entsprechenden Oelen, oder aber auch mit Soda.

Bevor Zedernholz in Stück-, Spahn- oder Pulverform mit Oelen anderer natürlicher Stoffe gemischt wird, kann es vorteilhaft sein das Zedernholz teilweise auszupressen und/oder auszutrocknen, um die Aufnahme des Fremdöls zu verbessern.

Die oben beschriebenen Lufterfrischer, insbesonders jene mit Aktivkohle und/oder Zedernholz sind z.B. geeignet für die Verwendung in Schränken inklusive Schuh-, Küchen und Kühlschränken, ferner Schubladen, Kehrichteimern und/oder Kehrichteimerfächern sowie allgemein im Wohn- und Toilettenbereich.

### Beispiel 1: Lufterfrischer

Ein Lufterfrischer mit folgenden Dimensionen

| | |
|---|---|
| Länge: | 30 mm |
| Breite: | 25 mm |
| Höhe: | 45 mm |

der 20 g Aktivkohle in Zylinderform (Länge 3-6 mm, Durchmesser 2-4 mm, Oberfläche von mehreren tausend m²/g) enthält, sowie 1-20 g Zedernholz mit einer Oberfläche von mehreren hundert m²/g hat in einem Volumen von 0.01-0.5 m³ Luft eine Lebensdauer von ca. 3-6 Monaten.

### Beispiel 2: natürliches Mottenvertilgungsmittel

Insektenart: Kleidermotte (Tineola bisselliella), frisch geschlüpft
Anzahl: 25 Tiere
Versuchsdurchführung: 25 Motten wurden in ein Testgefäss gegeben. Ein frisch angeschliffenes Stück Holz der "Eastern Aromatic Red Cedar" wurde zugegeben. Nach ca. 48 Stunden legten die Motten Eier, vorzugsweise auf dem Holz selbst oder in dessen unmittelbarer Nähe. Nur aus wenigen Eiern schlüpften Larven aus. Alle Larven starben.

## Patentansprüche

1. Lufterfrischer umfassend ein Sorptionsmittel, dadurch gekennzeichnet, dass er mindestens in Hauptrichtung der Luftströmung durchströmbar ist.

2. Lufterfrischer gemäss Anspruch 1, dadurch gekennzeichnet, dass er allseitig durchströmbar ist.

3. Lufterfrischer gemäss Anspruch 1 oder 2, dadurch gekennzeichnet, dass er einen Behälter enthaltend ein Sorptionsmittel aufweist.

4. Lufterfrischer gemäss Anspruch 3, dadurch gekennzeichnet, dass der Behälter ein mindestens in Hauptrichtung der Luftströmung durchströmbares, insbesonders ein allseitig durchströmbares Kunststoffgehäuse aufweist.

5. Lufterfrischer gemäss Anspruch 3 oder 4, dadurch gekennzeichnet, dass der Behälter würfelförmig ist und Mittel zu dessen Fixierung auf einer Ecke oder insbesondere auf einer Kante aufweist.

6. Lufterfrischer gemäss einem der Ansprüche 3 bis 5, dadurch gekennzeichnet, dass der Behälter aus einem Kunststoffgehäuse mit grossen Oeffnungen besteht, die mit einer porösen Filtermatte verschlossen sind.

7. Lufterfrischer gemäss einem der Ansprüche 1 bis 6, dadurch gekennzeichnet, dass er als Sorptionsmittel Aktivkohle und/oder Zedernholz enthält.

8. Lufterfrischer gemäss Anspruch 7, dadurch gekennzeichnet, dass das Zedernholz Holz der "Eastern Aromatic Red Cedar" ist.

9. Verwendung von Zedernholz, insbesonders in Spahnform, zur Geruchsbindung und/oder zum Fernhalten von Flöhen, Zecken, Stechmücken und/oder Küchenschaben, insbesonders in Lufterfrischern gemäss einem der Ansprüche 1 bis 8.

10. Verwendung gemäss Anspruch 9, dadurch gekennzeichnet, dass das Zedernholz Holz der "Eastern Aromatic Red Cedar" ist.

11. Verwendung gemäss Anspruch 9 oder 10, dadurch gekennzeichnet, dass das Zedernholz zur Geruchsbindung, insbesondere in Lufterfrischern gemäss einem der Ansprüche 1-8 verwendet wird.

12. Verwendung gemäss einem der Ansprüche 9 bis 11, dadurch gekennzeichnet, dass das Zedernholz gemeinsam mit einem weiteren Sorptionsmittel, insbesonders Aktivkohle und/oder Soda, verwendet wird.

13. Verwendung gemäss einem der Ansprüche 9 bis 12, dadurch gekennzeichnet, dass das Zedernholz teilweise ausgepresst oder getrocknet und mit einem natürlichen Oel getränkt ist.

14. Verwendung gemäss einem der Ansprüche 9 bis 13 in Katzenstreu.

15. Verwendung von Holz der "Eastern Aromatic Red Cedar" als Mottenvertilgungsmittel.
